(19) Europäisches Patentamt
European Patent Office
Office européen des brevets

(11) Numéro de publication: **0 157 661**
**B1**

(12) # FASCICULE DE BREVET EUROPÉEN

(45) Date de publication du fascicule du brevet: **24.02.88**

(51) Int. Cl.⁴: **C 07 C 119/042**, C 07 C 118/02, C 07 D 245/02

(21) Numéro de dépôt: **85400250.8**

(22) Date de dépôt: **14.02.85**

(54) **Diisocyanates 1-10 décane et leurs procédés de fabrication.**

(30) Priorité: **02.03.84 FR 8403253**

(43) Date de publication de la demande: **09.10.85 Bulletin 85/41**

(45) Mention de la délivrance du brevet: **24.02.88 Bulletin 88/8**

(84) Etats contractants désignés: **BE CH DE FR GB IT LI NL**

(56) Documents cité:
**DE-A-2 012 755**
**FR-A-1 447 612**
**FR-A-1 578 808**
**US-A-2 865 940**
**US-A-4 224 238**

(73) Titulaire: **ATOCHEM, 4 & 8, Cours Michelet La Défense 10, F-92800 Puteaux (FR)**

(72) Inventeur: **Kervennal, Jacques, 134, Rue E. Locard, F-69005 Lyon (FR)**
Inventeur: **Durual, Pierre, Les Essarts No 2 Chemin du Rossignol, F-69390 Vernaison (FR)**

(74) Mandataire: **Foiret, Claude, ATOCHEM Département Propriété Industrielle, F-92091 Paris la Défense 10 Cédex 42 (FR)**

**EP 0 157 661 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

L'invention concerne des diisocyanates aliphatiques de formule générale diisocyanates-1,10 décane, la chaîne alkyle pouvant être diversement substituée en positions 1 et 10. Ces isocyanates sont préparés par phosgénation des amines correspondantes et sont particulièrement intéressants dans la synthèse de polyuréthannes utilisés notamment dans l'élaboration de vernis et de peintures non jaunissantes.

Dans le brevet américain 4.224.238 sont décrits des produits de formule:

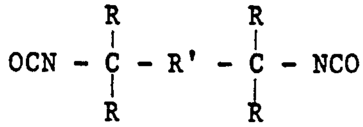

dans laquelle R représente un groupement alkyle et R' représente un groupement alkylène cyclique ou acyclique. De tels produits, selon ledit brevet, sont préparés par réaction d'un dihalogénure d'alkyle, dont chaque halogène est fixé sur un carbone tertiaire, avec un cyanate de métal alcalin. Or il n'est pas possible de trouver dans la littérature l'existence d'un quelconque dihalogénure d'alkyle dont chaque halogène soit fixé sur un carbone tertiaire pour la raison qu'un tel produit n'existe pas. Il en résulte que le procédé de préparation desdits produits enseigné par le brevet américain 4.224.238, dont aucun exemple d'illustration concrète n'est d'ailleurs donné, ne peut pas être mis en oeuvre et qu'en conséquence aucun desdits produits n'a été isolé.

Deux techniques de phosgénation peuvent être avantageusement mises en oeuvre pour préparer les diisocyanates objet de l'invention. L'une consiste à faire réagir à basse température l'amine avec le phosgène dans un solvant pour former du chlorure de carbamoyle, puis à élever la température pour décomposer celui-ci et récupérer l'isocyanate. L'autre consiste à synthétiser le chlorhydrate de l'amine, à l'isoler et à le faire réagir à chaud dans un intervalle de température de 120 à 190°C avec un courant de phosgène gazeux.

Les diisocyanates de l'invention correspondent à la formule générale (I) suivante:

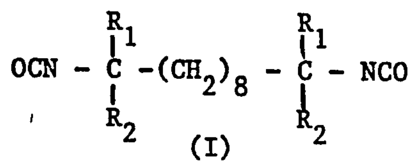

(I)

dans laquelle $R_1$ et $R_2$ sont identiques ou différents et où $R_1$ représente un radical alkyle ou cycloalkyle et $R_2$ un atome d'hydrogène, un radical alkyle ou cycloalkyle. Les groupements alkyles sont des radicaux à chaîne droite ou ramifiée ayant de préférence de 1 à 10 atomes de carbone; s'ils sont ramifiés, les substituants sont de préférence des groupements alkyles. On trouve ainsi de façon non limitative les radicaux méthyle, éthyle, n-propyle, isopropyle, n-,iso et tertiobutyle, n-pentyle, n-hexyle, n-heptyle, n-octyle et n-décyle. Les radicaux cycloalkyles contiennent de préférence de 5 à 10 atomes de carbone tels que les cyclopentyles, cyclohexyles, méthylcyclohexyles, cyclooctyles, etc...

Ces diisocyanates sont obtenus par phosgénation des diamines

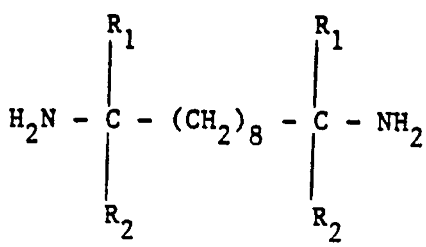

$R_1$ et $R_2$ correspondant aux définitions données précédemment. Ces diamines peuvent être obtenues par hydrogénation puis hydrogénolyse de diaza-1,2 cyclododécatriènes-1,5,9 résultant eux-mêmes de la cyclooligomérisation des azines et du butadiène.

L'oligomérisation du butadiène en cyclododécatriène est une réaction connue et décrite par exemple dans le brevet américain 3 239 574 avec comme catalyseur le tétrachlorure de titane et de triéthylaluminium. On peut également utiliser comme catalyseur des métaux de transition tels que le nickel ainsi que cité par B. BOGDANOVIC, P. HEIMBACH, M. KRÖNER, G. WILKE, E.G. HOFFMANN et J. BRANDT dans Liebigs Ann. Chem. 727, 143 (1969).

Plus récemment, des publications ont montré que l'introduction d'atomes d'azote dans le cycle était possible; les brevets français 2 189 408 et 2 292 698 et P. HEIMBACH et coll. dans Angew. Chem. Int. Ed. Engl. vol. 15, n° 1 p. 49 (1976) décrivent la cyclooligomérisation de deux molécules de butadiène avec une molécule d'azine pour former des diaza-1,2 cyclododécatriènes-1,5,9.

La cyclooligomérisation, en présence de catalyseur, des azines et du butadiène menant aux diaza-1,2 cyclododécatriènes-1,5,9 peut s'écrire

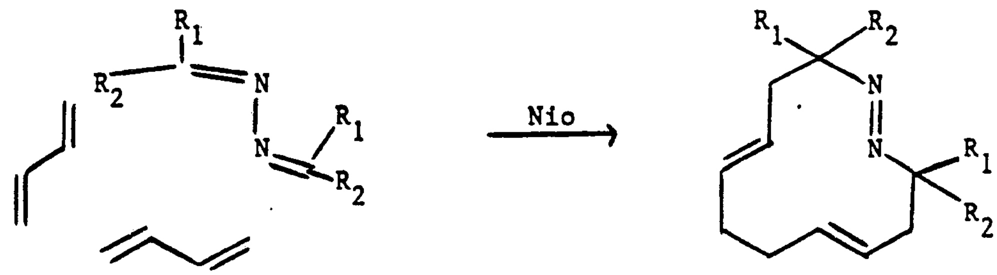


$R_1$ et $R_2$ correspondant toujours aux définitions données précédemment.

Cette réaction est réalisée en milieu inerte dans un solvant de préférence aromatique tel que benzène ou toluène, le catalyseur étant préparé "in situ" dans le solvant par réduction du bis (acétylacétonate) de nickel par le diéthyléthoxyaluminium en présence éventuellement d'un ligand phosphine.

L'hydrogénation puis l'hydrogénolyse du cycle en diamine se fait habituellement sous pression d'hydrogène dans un solvant organique, en présence d'un catalyseur. Suivant le composé, il peut être nécessaire d'opérer en une ou deux étapes en changeant éventuellement de catalyseur. Les doubles liaisons

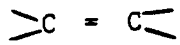

s'hydrogènent facilement à température modérée, entre 30 et 45° C. L'hydrogénation de la liaison N = N puis la coupure exigent des conditions opératoires plus sévères avec des températures supérieures à 140° C et des pressions d'hydrogène supérieure à 120 bars. On peut schématiser la réaction de façon suivante:

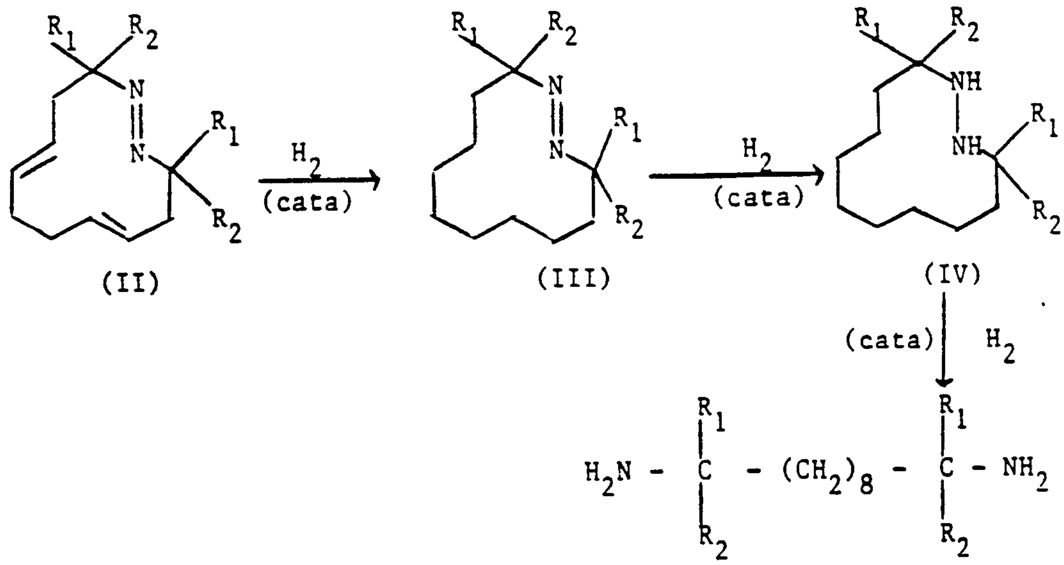


(II) (III) (IV)

(V)

L'intermédiaire (III) n'est pas isolé; quant au (IV) celà dépend de la nature des substituants $R_1$ et $R_2$. Si $R_2$ est différent de H, il est généralement préférable d'opérer en deux étapes comme précisé ci-dessus.

Les hydrogénations des diaza-1,2 cyclododécatriènes-1,5,9 et les hydrogénolyses des liaisons

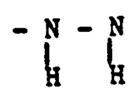

sont effectuées de façon connue, en autoclave, en présence de catalyseurs massiques tels que le nickel de Raney ou supportés tels que du palladium ou du rhodium déposés sur charbon ou alumine. Suivant la nature des substituants, il est préférable d'adapter la technique de l'hydrogénation. Ainsi, dans les cas où on réalise la condensation à l'aide d'une azine dérivée d'un aldéhyde, on peut opérer avec de bons rendements les différents stades d'hydrogénation en présence d'un seul catalyseur tel que du rhodium supporté, dans un solvant tel que le tertiobutanol. Il est préférable de respecter plusieurs paliers; on hydrogène ainsi en quelques minutes entre 30 et 40° C sous une pression d'hydrogène comprise entre 100 et 120 bars les doubles liaisons

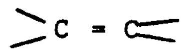

du cycle, puis on porte graduellement la température à 140-170°C sous une pression de 140 à 200 bars pendant plusieurs heures pour obtenir les diamines répondant à la formule (V) donnée ci-dessus, $R_2$ étant l'hydrogène. Dans les cas où on utilise une cétone comme matière première, il est recommandé d'opérer en deux étapes: dans un premier temps, on hydrogène, par exemple en présence de palladium déposé sur charbon, à 30-40°C sous 120 à 140 bars, les doubles liaisons

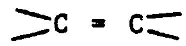

du cycle, puis on transforme, aux environs de 100°C sous 160 à 200 bars pendant plusieurs heures, la double liaison - N = N - en liaison

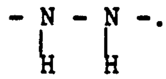

Après avoir isolé du milieu l'hydrazine cyclique ainsi obtenue, on la charge à nouveau dans le réacteur en présence d'ammoniac et d'un autre catalyseur métallique tel que le nickel de Raney; en opérant aux environs de 200°C sous 120 à 140 bars d'hydrogène pendant plusieurs heures, on obtient les diamines attendues.

Les azines correspondant à l'invention généralement obtenues par réaction d'une mole d'hydrazine sur deux moles d'aldéhyde ou de cétone selon la réaction:

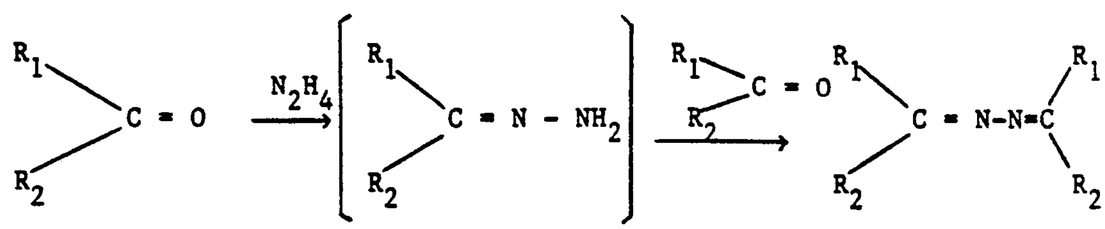


l'hydrazone intermédiaire n'étant généralement pas isolable.

Dans le cas où la réaction s'effectue à partir d'aldehyde on peut opérer selon la méthode décrite par A.N. KOST et I.I. GRANDBERG dans le Journal of General Chemistry (URSS) (1955), 25, 2017; (1956), 26, 1925, 2201 et 2905 et (1961), 31, 869. On coule à température inférieure à -10°C, de l'hydrate d'hydrazine en léger excès sur l'aldéhyde en solution dans l'éther. On laisse le mélange revenir à température ordinaire et on ajoute de la potasse en pastilles jusqu'à saturation. On sépare les deux couches et récupère la phase éthérée; l'azine est ensuite distillée. Dans le cas des cétones, celles-ci sont additionnées sur l'hydrate d'hydrazine à température inférieure à 20°C. On distille l'eau azéotropiquement après avoir rajouté du benzène, selon la méthode décrite par T. CURTIUS et K. KHUN dans le J. Prakt. Chem. (1891), 44, 92, 151, 543. On finit de sécher en ajoutant de la potasse puis récupère et distille la phase organique.

La cyclooligomérisation des azines et du butadiène peut être catalysée entre 50 et 95°C par du "nickel nu" préparé "in situ", c'est-à-dire du nickel au degré d'oxydation zéro obtenu par réduction d'un complexe de nickel II, tel que l'acétylacétonate de nickel, par un organo-aluminique comme le diéthyléthoxyaluminium en présence de butadiène. Habituellement le rapport molaire $\frac{\text{nickel}}{\text{azine}}$ peut varier entre 0,1 et 50 % et mieux entre 1 et 10 %.

Il est possible d'opérer la réduction en présence d'un ligand tel qu'une phosphine ou un phosphite capable de se coordiner sur le métal. On sait, en effet, qu'en catalyse homogène, la modification des paramètres électroniques ou stériques du ligand associé au métal est de nature à orienter la réaction vers des processus réactionnels différents. Divers ligands aliphatiques ou aromatiques peuvent être utilisés tels que la triméthylphosphine, la tricyclohexylphosphine, la triphénylphosphine, la tributylphosphine, le triphénylphosphite, le triméthylphosphite... Le rapport molaire $\frac{\text{ligand}}{\text{Ni}}$ peut varier entre 0,1 et 10.

Un facteur important dans l'orientation de la réaction est le rapport du butadiène sur l'azine mis en oeuvre. Il est possible de travailler avec la quantité stoéchiométrique de butadiene par rapport à l'azine, mais il est préférable d'opérer en présence d'un excès de butadiène. Cependant, l'excès ne doit pas être trop important afin de limiter la formation de sous produits tels que le vinylcyclohexène, le cyclooctadiène et le cyclododécatriène qui sont habituellement obtenus dans la cyclo-oligomérisation du butadiène. Dans certains cas, il peut se former également le dérivé tétraaza-1,2,5,6 cyclododécatriène-1,5,9 résultant de la condensation d'une mole de butadiène et de deux moles d'azine.

La phosgénation des diamines précédemment décrites permet d'obtenir les isocyanates de l'invention. Sans

être exclusives les deux techniques décrites ci-après donnent d'excellents résultats.

Selon la première on peut introduire la diamine dans le solvant contenant le phosgène dissous et maintenu à température inférieure à 20°C et de préférence entre 0 et 20°C. Il se forme immédiatement un précipité de chlorure de carbamoyle. On élève ensuite la température en respectant de préférence mais non nécessairement des paliers à des températures voisines de 60 et 100°C. On observe, à partir de 60°C, une solubilisation du précipité. On termine au reflux du mélange, sous atmosphère inerte pendant environ trois heures, puis refroidit, filtre pour éliminer le chlorhydrate formé et distille le solvant ainsi que le diisocyanate. On peut, dans ce cas, utiliser des solvants aliphatiques tels que le décane, la décaline, ou aromatiques tels que le monochlorobenzène, les dichlorobenzènes etc...

Selon la seconde on synthétise dans un premier temps le chlorhydrate de la diamine qu'on isole et sèche. On met ensuite le chlorhydrate en suspension dans un solvant tel que l'orthodichlorobenzène ou la tétraline et porte à la température du reflux, voisine de 180-185°C dans le cas des deux solvants cités. On introduit alors un courant de phosgène jusqu'à dissolution complète du précipité. On filtre, distille le solvant puis le diisocyanate.

Les structures des produits purifiés formés au cours des différentes étapes ont été analysées par résonance magnétique nucléaire du $^{1}H$ et du $^{13}C$. Les analyses courantes ont été effectuées par chromatographie en phase gazeuse en utilisant une colonne de 2 mètres remplie de phase silicone SE 30 ou, pour les amines, une colonne en verre de 2 mètres, remplie de support chromosorb W.N.A.W. 60-80 mesh (commercialisé par John Manville), imprégné à 5 % de KOH et 5 % d'Apiézon N (commercialisé par Apiézon Products Limited), en opérant en isotherme à 220°C. Les identifications des pics, lorsque c'était nécessaire, ont été faites par couplage avec la spectrométrie de masse.

Les exemples ci-dessous sont donnés à titre d'illustration de l'invention.

**Exemple 1**

Dans un réacteur de 2 litres refroidi à -20°C, on place 300 g d'acétaldéhyde dans 125 ml d'éther. On verse goutte à goutte en maintenant la température aux environs de -15°C, 136 g d'hydrate d'hydrazine. On laisse ensuite la température revenir à 25°C puis ajoute 140 g de potasse en pastilles. On laisse décanter et extrait la phase éthérée qu'on sèche et distille. On obtient 131 g d'acétaldazine distillant à 98°C.

On introduit dans un réacteur de 2 litres 9,7 g d'acétylacétonate de nickel déshydraté (0,038 mole) 9,8 g de triphénylphosphine (0,038 mole) qu'on solubilise dans 180 ml de benzène anhydre. On met sous argon et refroidit en dessous de 10°C. On injecte alors 9,9 g de diéthyl-éthoxy aluminium (0,076 mol.). La solution vire du vert au marron et on laisse revenir à température ambiante, puis on introduit, toujours sous argon un mélange de 63 g d'acétaldazine (0,75 mole) et de 202 g de butadiène (3,7 moles) solubilisés dans 750 ml de benzène anhydre. On isole le réacteur et le porte à 50°C pendant 12 heures. On évapore ensuite le solvant, puis précipite le catalyseur à l'aide d'hexane. Après filtration, on distille le mélange sous 1 333 Pa et récupère 63,2 g de diméthyl-3,12 diaza-1,2 cyclododécatriène-1,5,9 distillant entre 108 et 110°C (rendement 44 %).

Dans un autoclave en inox de 300 ml on introduit 15 g du cycle de condensation dans 105 ml de tertiobutanol et on ajoute 1,5 g de catalyseur au rhodium à une concentration de 5 % sur de l'alumine (commercialisé par ENGELHARD). Après balayage du réacteur à l'azote, on intoduit 120 bars d'hydrogène et chauffe à 40°C. On laisse réagir pendant 30 minutes. On monte ensuite jusqu'à 145°C sous 135 bars en respectant des paliers à 75 et 100°C. On laisse réagir 20 heures à 145°C puis refroidit, filtre et distille. On récupère 13 g de diamino-1,10 diméthyl-1,10 décane (rendement: 83 %) qu'on dissout dans 11 ml de méthanol. On ajoute goutte à goutte 12,2 ml d'HCl à 37 % en limitant la température à 30°C. On finit de précipiter le chlorhydrate en ajoutant lentement 140 ml d'acétone. On récupère et sèche le précipité (16,5 g). On introduit ensuite celui-ci dans un réacteur de 100 ml contenant 70 ml de tétraline séchée sur sulfate de sodium. On agite, met sous azote et chauffe. A 150°C on fait passer un courant de 1 l/h de phosgène. A 175°C, le milieu s'homogénéise et prend une coloration beige qui vire au marron à 180°C. On reste en palier pendant une heure sous balayage de phosgène jusqu'à ce que le milieu devienne totalement limpide, puis on fait passer un courant d'azote et refroidit. On distille la tétraline, puis l'isocyanate (146°C sous 400 Pa); on recueille 12,35 g de liquide incolore correspondant au diisocyanate-1,10 diméthyl-1,10 décane (teneur NCO de 2 équivalents par mole). Rendement: 81 % par rapport au chlorhydrate.

Analyse élémentaire:

théorique - C: 66,63 % - H: 9,58 % - N: 11,10 %

trouvé - C: 66,52 % - H: 9,73 % - N: 10,86 %

caractéristiques en R.M.N. $^{13}C$ (déplacements chimiques en ppm par rapport au solvant $CDCl_3$ à 77 ppm:

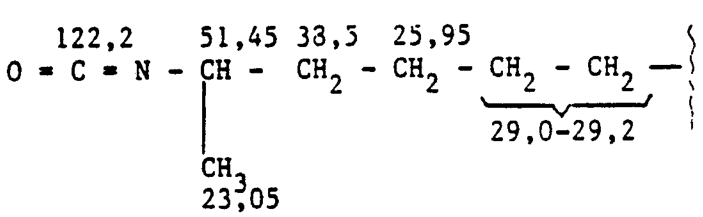

**Exemple 2**

On prépare de l'isobutyraldazine en faisant réagir de l'isobutyraldéhyde sur de l'hydrate d'hydrazine suivant les conditions opératoires décrites dans l'exemple 1. On réalise ensuite la réaction de cyclooligomérisation de l'azine sur le butadiène en utilisant un rapport molaire de 1/3 et en opérant comme dans l'exemple 1 (1 at. g de Ni pour 20 moles d'azine). On hydrogène dans 100 ml de tert. butanol, 14 g de diisopropyl-3,12 diaza-1,2 cyclododécatriène-1,5,9 en autoclave de 300 ml, en présence de 1,4 g de catalyseur au rhodium déposé à 5 % sur alumine. On laisse réagir pendant 30 minutes sous 100 bars d'hydrogène à 30° C, puis on maintient un palier à 160° C sous 200 bars d'hydrogène pendant 25 heures. On chlorhydrate et phosgène de la même façon que dans l'exemple 1, 12 g du diamino-1,10 diisopropyl-1,10 décane ainsi obtenu. On récupère 13,2 g de diisocyanate-1,10 diisopropyl-1,10 décane (Rendement de phosgénation: 92 % par rapport à l'amine) ayant une teneur en fonctions NCO de 1,85 équivalent par mole et présentant les caractéristiques suivantes en R.M.N. du $^{13}C$ (déplacements chimiques exprimés par rapport à $CDCl_3$ à 77 ppm):

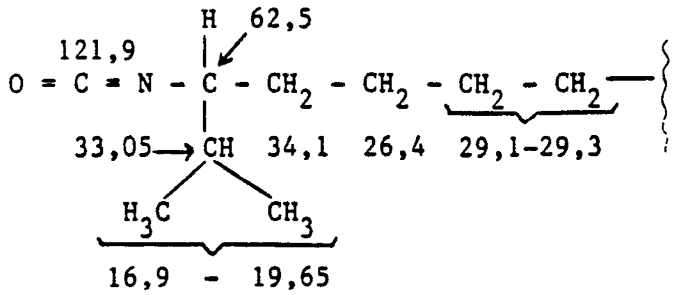

**Exemple 3**

On synthétise de la propionaldazine par réaction de propionaldéhyde sur de l'hydrate d'hydrazine en opérant comme dans l'exemple 1. On effectue ensuite la réaction de cyclooligomérisation, suivant les conditions décrites dans l'exemple 1, en faisant réagir l'azine sur le butadiène dans un rapport 1/3 en présence de nickel préparé par réduction d'acétylacétonate de nickel (1 at.g. de Ni pour 20 moles d'azine) et de triphénylphosphine. On hydrogène dans 100 ml de tertiobutanol, 12,15 g de diéthyl-3,12 diaza-1,2 cyclododécatriène-1,5,9 en autoclave de 300 ml, en présence de 1,3 g de catalyseur au rhodium déposé à 5 % sur alumine. On fait réagir pendant 30 minutes à 45° sous 130 bars d'hydrogène, puis pendant 11h30 à 185° C sous 150 bars. La diamine (8,71 g) est phosgénée, sous forme du chlorhydrate, comme dans l'exemple 1 pour conduire à 9,6 g (Rend. 90 %) d'isocyanate brut. La distillation de ce dernier conduit au diisocyanate-1,10 diéthyl-1,10 décane, liquide incolore, ayant une teneur de 2 fonctions NCO par mole et les caractéristiques suivantes en R.M.N. du $^{13}C$ (déplacements chimiques par rapport à $CDCl_3$ à 77 ppm):

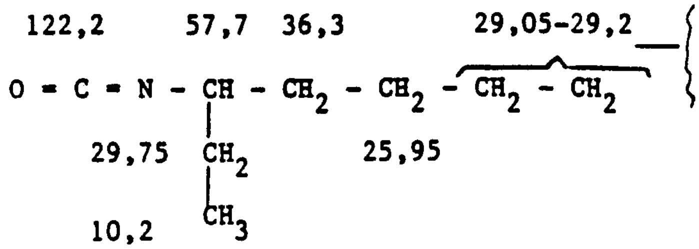

Analyse élémentaire:
théorique: C = 70,09 %; H = 10,45 %; N = 9,08 %
trouvé: C = 69,78 %; H = 10,25 %; N = 9,40 %

**Exemple 4**

On prépare de l'hexahydrobenzaldazine par réaction de l'hexahydrobenzaldéhyde sur l'hydrate d'hydrazine suivant le mode opératoire décrit dans l'exemple 1. Le dicyclohexyl-3,12 diaza-1,2 cyclododécatriène-1,5,9 est ensuite synthétisé en faisant réagir l'hexahydrobenzaldazine sur le butadiène dans un rapport molaire 1/3 en présence de nickel et de triphénylphosphine suivant la description présentée dans l'exemple 1 (1 at.g. de Ni pour 20 moles d'azine). On obtient un produit solide blanc dont on hydrogène 10 g en autoclave de 300 ml dans 100 ml de tertiobutanol en présence de 1 g de catalyseur au rhodium déposé à 5 % sur l'alumine; on opère en

deux phases, tout d'abord à 40°C pendant 30 minutes sous 150 bars d'hydrogène, puis à 216°C pendant 2 heures sous 157 bars. La diamine, liquide incolore, est chlorhydratée (9,4 g) puis phosgénée suivant le procédé utilisé dans l'exemple 1. On récupère 8,6 g de diisocyanate, d'aspect huileux, ayant une teneur NCO de 1,95 équivalent/mole (pureté 97,5 %), et présentant les caractéristiques suivantes en R.M.N. du $^{13}C$ (déplacements chimiques par rapport à $CDCl_3$ à 77 ppm):

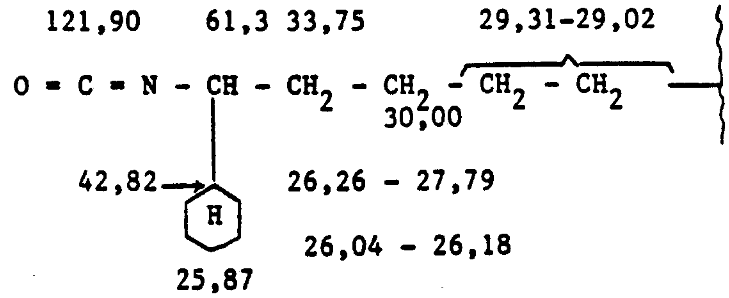


Analyse élémentaire:
théorique: C = 74,18 %; H = 10,37 %; N = 7,21 %
trouvé: C = 74,07 %; H = 10,59 %; N = 7,06 %

**Exemple 5**

On coule 190 g d'acétone (3,3 moles), goutte à goutte, sur 100 g d'hydrate d'hydrazine (2 moles) en maintenant la température en dessous de 17°C. On laisse la température remonter à 25°C en fin d'addition, puis on ajoute 100 ml de benzène anhydre et élimine l'eau du milieu réactionnel par distillation azéotropique. On finit de sécher le mélange en ajoutant 20 g de potasse en pastilles, puis sépare les deux couches, récupère la phase organique et distille à pression atmosphérique. On récupère 146 g d'acétazine distillant à 129-131°C.

On fait réagir 76 g d'acétazine (0,678 mole) avec 108 g de butadiène (2 moles) en présence de nickel et de triphénylphosphine en opérant de la même façon que dans l'exemple 1. On récupère 60,5 g de tétraméthyl-3,3,12,12 diaza-1,2 cyclododécatriène-1,5,9 distillant à 100°C sous 400 Pa. On en introduit 20 g dans un autoclave de 300 ml contenant 110 ml de monoéthyléther de l'éthylène glycol et 0,9 g de palladium déposé à 5 % sur charbon (ENGELHARD). On introduit 136 bars d'hydrogène et laisse réagir à 30°C pendant 30 minutes, puis porte à 100°C sous 180 bars d'hydrogène pendant 8h30. Après refroidissement, on détend, vide le réacteur et filtre à 40°C. On récupère 18,5 g de l'hydrazine cyclique correspondante qui précipite. On en dissout 10 g dans 60 ml de tert.butanol, et introduit la solution dans l'autoclave avec 5 g de nickel de Raney fraichement préparé et 13 g de $NH_3$. En montant progressivement la température jusqu'à 200°C, on fait réagir pendant 16 heures sous 120 bars d'hydrogène. Après refroidissement, on filtre et évapore le solvant, puis on solubilise le résidu de distillation dans 50 ml d'orthodichlorobenzène et introduit la solution dans un réacteur à 10°C contenant 40 ml de phosgène dissous dans 50 ml d'orthodichlorobenzène. Le mélange réactionnel prend une coloration jaune. On chauffe à 140°C avec un palier d'une heure après avoir respecté deux paliers d'une heure l'un à 60°C l'autre à 100°C. La coloration jaune initiale vire au marron. Après balayage à l'azote et refroidissement, on filtre, récupère le filtrat et évapore le solvant, puis distille le diisocyanate (147-150°C sous 266 Pa). On recueille 2 g de diisocyanate-1,10 tétraméthyl-1,1,10,10 décane (teneur en NCO: 1,9 équivalent par mole).

Analyse élémentaire:
théorique: C = 68,53 %; H = 10,06 %; N = 9,99 %
trouvé: C = 69,71 %; H = 10,47 %; N = 9,75 %
caractéristiques en R.M.N. du $^{13}C$ ($CDCl_3$).

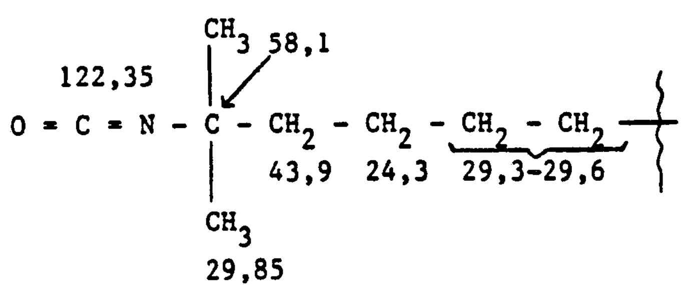

**Revendications**

1. Diisocyanates-1,10 dècane répondant à la formule

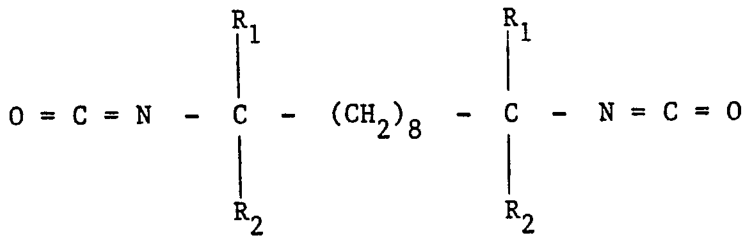

dans laquelle $R_1$ et $R_2$ sont identiques ou différents et où
$R_1$ représente un radical alkyle ou cycloalkyle
et $R_2$ représente un atome d'hydrogène, un radical alkyle ou cycloalkyle.

2. Diisocyanates selon la revendication 1 caractérisés en ce que les radicaux alkyles sont des radicaux à chaîne droite ou ramifiée ayant de 1 à 10 atomes de carbone.

3. Diisocyanates selon la revendication 2 caractérisés en ce que les ramifications des radicaux alkyles ramifiés sont des groupements alkyles.

4. Diisocyanates selon la revendication 1 caractérisés en ce que les radicaux cycloalkyles contiennent de 5 à 10 atomes de carbone.

5. Procédé de fabrication des diisocyanates des revendications 1 à 4 caractérisé en ce qu'on soumet à phosgénation les diamino-1,10 décane de formule

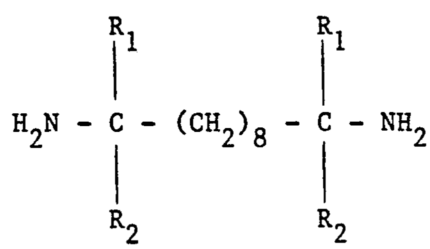

$R_1$ et $R_2$ représentant les radicaux tels que définis de 1 à 4.

6. Procédé selon la revendication 5 caractérisé en ce qu'on met en contact la diamine avec le phosgène en milieu solvant à une température inférieure à 20°C puis à faire réagir à la température du reflux du mélange.

7. Procédé selon la revendication 5 caractérisé en ce qu'on transforme la diamine en chlorhydrate sur lequel on fait réagir le phosgène en milieu solvant à la température de reflux du mélange.

8. Procédé selon l'une des revendications 5 à 7 caractérisé en ce que les diamino-1,10 décane sont obtenus par hydrogénation puis hydrogénolyse de diaza-1,2 cyclododécadiène-1,5,9.

9. Procédé selon la revendication 8 caractérisé en ce que les diaza-1,2 cyclododécadiènes-1,5,9 résultent de la cyclooligomérisation des azines et du butadiène.

**Patentansprüche**

1. Diisocyanato-1,10-decane der Formel

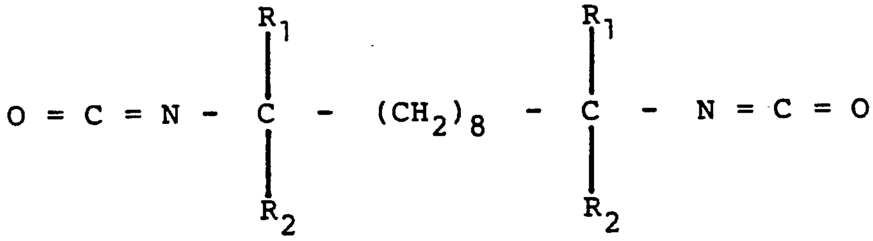
,

worin $R_1$ und $R_2$ gleich oder unterschiedlich sein können und $R_1$ eine Alkyl- oder Cycloalkylgruppe bedeuten und $R_2$ zusätzlich auch ein Wasserstoffatom sein kann.

2. Diisocyanato-1,10-decane nach Anspruch 1, bei denen die Alkylgruppen geradkettig oder verzweigt sein können und 1 bis 10 Kohlenstoffatome enthalten.

3. Diisocyanato-1,10-decane nach Anspruch 2, bei denen die Verzweigungen der verzweigten Alkylgruppen Alkylgruppen bilden.

4. Diisocyanato-1,10-decane nach Anspruch 1, in denen die Cycloalkylgruppen 5 bis 10 Kohlenstoffatome enthalten.

5. Verfahren zur Herstellung der Diisocyanato-1,10-decane nach Anspruch 1 bis 4, dadurch gekennzeichnet, daß man Diamino-1,10-decan der Formel

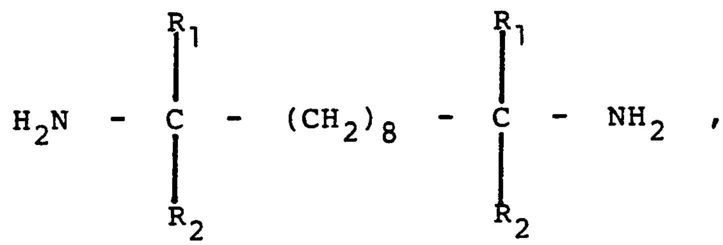

worin $R_1$ und $R_2$ obige Bedeutung haben, mit Phosgen behandelt.

6. Verfahren nach Anspruch 5, dadurch gekennzeichnet, daß man das Diamin mit Phosgen in einem Lösungsmittel bei einer Temperatur unter 20°C zusammenbringt und die Reaktion unter rückfließendem Sieden durchführt.

7. Verfahren nach Anspruch 5, dadurch gekennzeichnet, daß man das Diamin in das Chlorhydrat überführt und dieses dann in einem Lösungsmittel mit Phosgen bei der Temperatur des rückfließenden Siedens umsetzt.

8. Verfahren nach Anspruch 5 bis 7, dadurch gekennzeichnet, daß das verwendete Diamino-1,10-decan erhalten worden ist durch Hydrieren und dann Hydrogenolyse von Diaza-1,2-cyclododecadien-1,5,9.

9. Verfahren nach Anspruch 8, dadurch gekennzeichnet, daß das Diaza-1,2-cyclododecadien-1,5,9 durch Cyclooligomerisierung von Azinen und Butadien erhalten worden ist.

**Claims**

1. 1,10-Diisocyanato-decanes corresponding to the formula

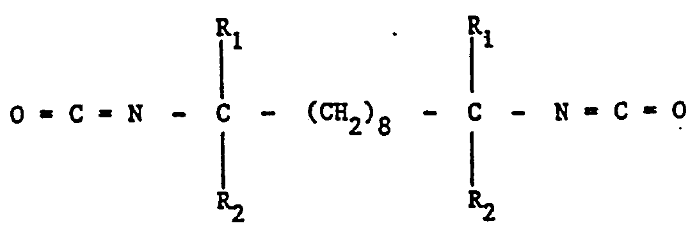

in which $R_1$ and $R_2$ are identical or different and where $R_1$ represents an alkyl or cycloalkyl radical and $R_2$ represents a hydrogen atom or an alkyl or cycloalkyl radical.

2. Diisocyanates according to Claim 1, characterized in that the alkyl radicals are straight-chain or branched chain radicals having from 1 to 10 carbon atoms.

3. Diisocyanates according to Claim 2, characterized in that the branches of the branched alkyl radicals are alkyl groups.

4. Diisocyanates according to Claim 1, characterized in that the cycloalkyl radicals contain from 5 to 10 carbon atoms.

5. Process for the manufacture of the diisocyanates of Claims 1 to 4, characterized in that the 1,10-diamino-decanes of the formula

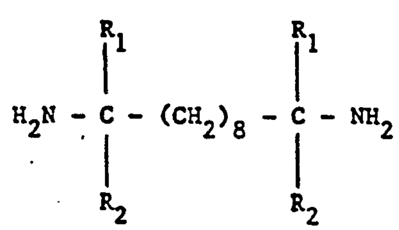

where $R_1$ and $R_2$ represent the radicals as defined from 1 to 4, are subjected to phosgenation.

6. Process according to Claim 5, characterized in that the diamine is brought into contact with phosgene in a solvent medium at a temperature below 20°C and reaction is then carried out at the reflux temperature of the mixture.

7. Process according to Claim 5, characterized in that the diamine is converted to a hydrochloride, which is reacted with phosgene in a solvent medium at the reflux temperature of the mixture.

8. Process according to one of Claims 5 to 7, characterized in that the 1,10-diamino-decanes are obtained by hydrogenation followed by hydrogenolysis of 1,2-diaza-1,5,9-cyclododecadiene.

9. Process according to Claim 8, characterized in that the 1,2-diaza-1,5,9-cyclododecadienes result from the cyclooligomerization of azines and butadiene.